(19) Europäisches Patentamt / European Patent Office / Office européen des brevets

(11) **EP 3 679 964 A1**

(12) **EUROPEAN PATENT APPLICATION**
published in accordance with Art. 153(4) EPC

(43) Date of publication:
**15.07.2020  Bulletin 2020/29**

(21) Application number: **18852826.9**

(22) Date of filing: **07.09.2018**

(51) Int Cl.:
*A61M 1/36* *(2006.01)*    *B01J 20/26* *(2006.01)*
*B01J 20/28* *(2006.01)*

(86) International application number:
**PCT/JP2018/033129**

(87) International publication number:
**WO 2019/049962 (14.03.2019 Gazette 2019/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **08.09.2017   JP 2017173122**

(71) Applicants:
  • **Toray Industries, Inc.**
    **Tokyo 103-8666 (JP)**
  • **National University Corporation Shiga University of Medical Science**
    **Otsu-shi**
    **Shiga 520-2192 (JP)**

(72) Inventors:
  • **UENO Yoshiyuki**
    **Otsu-shi**
    **Shiga 520-8558 (JP)**
  • **KASUYA Junichi**
    **Otsu-shi**
    **Shiga 520-8558 (JP)**
  • **ARAKANE Toru**
    **Tokyo 103-8666 (JP)**
  • **MATSUNAGA Ryo**
    **Otsu-shi**
    **Shiga 520-8558 (JP)**
  • **SEKIYA Yumiko**
    **Otsu-shi**
    **Shiga 520-8558 (JP)**
  • **UEDA Yuji**
    **Osaka-shi**
    **Osaka 545-0053 (JP)**
  • **TERAMOTO Kazuo**
    **Otsu-shi**
    **Shiga 520-2192 (JP)**
  • **OGASAWARA Kazumasa**
    **Otsu-shi**
    **Shiga 520-2192 (JP)**

(74) Representative: **Kador & Partner PartG mbB**
    **Corneliusstraße 15**
    **80469 München (DE)**

(54) **IMMUNOSUPPRESSIVE LEUKOCYTE ADSORPTION MATERIAL AND ADSORPTION COLUMN**

(57)    An objective of the present disclosure is to provide an adsorption material that can selectively adsorb immunosuppressive leukocyte. The present disclosure provides an adsorption material for immunosuppressive leukocyte. The adsorption material includes a water-insoluble carrier to which at least one nitrogen-containing compound selected from a polyamine represented by a predetermined formula and aliphatic amines represented by predetermined formulae are bound. A form of the water-insoluble carrier is a fiber or a particle, a diameter of the fiber or the particle is 15 to 50 $\mu$m, and an arithmetic mean roughness of a surface of the water-insoluble carrier is 0.1 to 3.0 $\mu$m.

**EP 3 679 964 A1**

# Fig. 1

**Description**

Technical Field

The present disclosure relates to an adsorption material for immunosuppressive leukocyte, and an adsorption column including the same.

Background Art

It has been becoming clear that cancers closely relate to immunity. Recently, it has been reported that immunosuppressive blood components rise in many advanced cancers.

A leucocyte that is deeply involved to an immune function is one of the blood components, and is classified into a lymphocyte, a granulocyte or a monocyte. Each of the leukocytes is further subdivided, and for example, the lymphocyte is classified into a T-cell, a B-cell, a natural killer cell, and the like.

Recently, as one of the molecules that is involved in a development of a cancer, it has been becoming clear that an enhancement of an immunosuppressive leukocyte, such as a LAP positive T-cell, protects cancer cells from an attack by an immune system to lead to the cancer progresses. It should be noted that a LAP means a Latency Associated Peptide, and is a protein having a molecular weight of approximately 75000. A LAP positive leukocyte is a leukocyte bound to the LAP, and is known as an immunosuppressive leukocyte.

While as a method for treating cancers, there has been developed medicines that block an immunosuppressive signal transmitted from cancer cells like a checkpoint antibody, cases of contractions of autoimmune diseases caused by side effects of the medicines are also recognized.

In order to enhance immune strength while reducing the side effects, a cell therapy that attempts to eliminate cancers by leukocytes of a patient himself/herself has been performed. A representative example is a dendritic cell infusion therapy that treats cancers by extracorporeally stimulating dendritic cells of a patient with a cancer antigen and then returning the dendritic cells to the patient to induce cancer-specific killer cells (cytotoxic T lymphocyte). However, this therapy currently has not reached a sufficiently satisfying therapeutic effect, and one of the reasons is presumed to be an enhanced immunosuppressive system.

Meanwhile, if the immunosuppressive leukocyte can be removed, the signal for protecting the cancer cells from the immune system is deactivated, and immune strength of a patient is enhanced. Therefore, regression of tumor and suppression of cancer progression can be expected.

Patent Literature 1 discloses a filter that is characterized in a fiber diameter, a bulk density, and the like of a nonwoven fabric as a material that removes leukocytes, and specifically discloses a leukocyte removal filter formed of a nonwoven fabric having a fiber diameter of less than 3 $\mu$m and a bulk density of more than 0.15 g/cm$^3$ and 0.50 g/cm$^3$ or less.

Patent Literature 2 discloses an adsorption material that is characterized in fiber diameter, surface area, and the like, and specifically discloses a cell adsorption column formed by being filled with an adsorbent including fibers with fiber diameters of 0.5 to 10 $\mu$m, and having surface areas of 0.5 m$^2$ or more and less than 10 m$^2$, in which adsorbent-filled volumes are 100 ml or less.

Patent Literature 3 discloses an adsorption material that is characterized in a ligand structure of an amino group, a content of the amino group, and the like.

Citation List

Patent Literature

Patent Literature 1: JP 60-193468 A
Patent Literature 2: WO2008/038785
Patent Literature 3: WO2012/133399

Summary of Invention

Technical Problem

However, the filter in Patent Literature 1 removes the leukocytes by filtering, and therefore, all kinds of leukocytes are removed, thereby causing a difficulty to selectively remove the immunosuppressive leukocyte. The adsorption material in Patent Literature 2 adsorbs cells using phagocytic activities of the granulocyte and the monocyte, and therefore, there is a possibility that it is difficult to selectively remove the LAP positive leukocyte, such as the LAP positive T-cell. The adsorption material in Patent Literature 3 is still required to improve in terms of selectivity of the immunosuppressive leukocyte.

For the above-mentioned reasons, it is desired to develop a technique that can selectively adsorb and remove the immunosuppressive leukocyte (in particular, the LAP positive leukocyte).

Therefore, an objective of the present disclosure is to provide an adsorption material that selectively adsorbs an immunosuppressive leukocyte.

Solution to Problem

The inventors unexpectedly found that containing a polyamine residue or an aliphatic amine residue in a water-insoluble carrier in a fiber form or a particle form, and furthermore, setting a diameter and an arithmetic mean roughness of the fiber or the particle to be in predetermined ranges ensures selectively adsorbing the immunosuppressive leukocyte (in particular, the LAP positive leukocyte), and thus have completed the present disclosure.

Exemplary aspects of the embodiment are described below.

[1] An adsorption material for immunosuppressive leukocyte, the adsorption material including

a water-insoluble carrier to which at least one nitrogen-containing compound is bound, the nitrogen-containing compound being selected from a polyamine represented by following Formula (1), a primary aliphatic amine represented by following Formula (2), and a secondary aliphatic amine represented by Formula (3),
wherein the water-insoluble carrier has a form of fiber or particle, the fiber or the particle has a diameter of 15 to 50 $\mu$m, and the water-insoluble carrier has a surface with an arithmetic mean roughness of 0.1 to 3.0 $\mu$m.

$$R^1R^2N\text{-}X\text{-}NR^3R^4 \ldots \qquad \text{Formula (1)}$$

[In Formula (1), X is a saturated or unsaturated aliphatic hydrocarbon group having 2 to 20 carbon atoms, or a heteroatom-containing carbon chain in which 1 to 5 carbon atoms of a saturated or unsaturated aliphatic hydrocarbon group having 3 to 20 carbon atoms are replaced with a nitrogen atom, a hydrogen atom that bonds to the nitrogen atom may be replaced with an alkyl group that may have an amino group, and $R^1$ to $R^4$ are each independently a hydrogen atom or an alkyl group.]

$$NH_2R^5 \ldots \qquad \text{Formula (2)}$$

[In Formula (2), $R^5$ is a saturated or unsaturated aliphatic hydrocarbon group having 1 to 12 carbon atoms.]

$$NHR^6R^7 \ldots \qquad \text{Formula (3)}$$

[In Formula (3), $R^6$ and $R^7$ are each independently a saturated or unsaturated aliphatic hydrocarbon group having 1 to 12 carbon atoms.]

[2] The adsorption material according to [1],
wherein the nitrogen-containing compound comprises the polyamine represented by the Formula (1).
[3] The adsorption material according to [1] or [2],
wherein the nitrogen-containing compound binds to the water-insoluble carrier via a linker.
[4] The adsorption material according to any one of [1] to [3],
wherein the water-insoluble carrier has a form of fiber.
[5] The adsorption material according to any one of [1] to [4],
wherein the immunosuppressive leukocyte is a LAP positive lymphocyte or a LAP positive monocyte.
[6] An adsorption column including the adsorption material according to any one of [1] to [5].
[7] The adsorption column according to [6],
wherein the adsorption column is used for a blood purification therapy.

Advantageous Effects of Invention

The present disclosure ensures providing an adsorption material that can selectively absorb and remove an immunosuppressive leukocyte (in particular, a LAP positive lymphocyte and a LAP positive monocyte).

Brief Description of Drawings

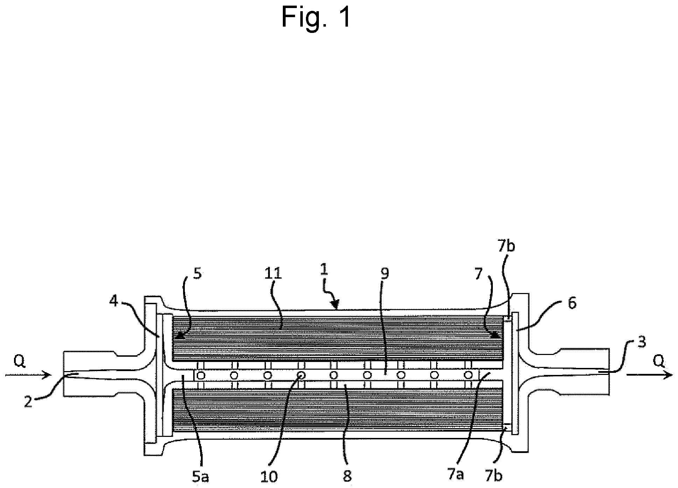

Fig. 1 is a vertical cross-sectional view of an exemplary radial flow type adsorption column.

Description of Embodiments

The following describes an embodiment further in details. It should be understood that throughout the entire Description, the expression as a singular form also includes a concept of its plural form unless otherwise stated. Accordingly, articles of a singular form (for example, in the case of English, "a," "an," and "the") should be understood as including the concept of its plural form unless otherwise stated. Terms used in the Description should be understood as used with meanings usually used in the technical field unless otherwise stated. Accordingly, unless otherwise defined differently, all the technical terms and the science and technology terms used in the Description have the same meanings as terms generally understood by a person skilled in the art pertaining to the present disclosure. In the case of inconsistency, the Description (including definition) has priority.

An adsorption material according to the embodiment relates to an adsorption material for adsorbing an immunosuppressive leukocyte. The adsorption material according to the embodiment includes a water-insoluble carrier to which at least one nitrogen-containing compound selected from a polyamine represented by Formula (1), a primary aliphatic amine represented by Formula (2), and a secondary aliphatic amine represented by Formula (3) is bound. In the adsorption material according to the embodiment, the water-insoluble carrier has a form of fiber or particle, the fiber or the particle has a diameter of 15 to 50 $\mu$m, and the water-insoluble carrier has a surface with an arithmetic mean roughness of 0.1 to 3.0 $\mu$m.

The adsorption material of the embodiment can selectively adsorb the immunosuppressive leukocyte. The adsorption material of the embodiment preferably has an excellent adsorption rate for the immunosuppressive leukocyte.

When it is used in the Description, a "nitrogen-containing compound residue" means a group obtained by directly or indirectly binding the nitrogen-containing compound to the water-insoluble carrier. Similarly, a "polyamine residue" means a group obtained by directly or indirectly binding the polyamine represented by Formula (1) to the water-insoluble carrier, and an "aliphatic amine residue" means a group obtained by directly or indirectly binding the aliphatic amine represented by Formula (2) or Formula (3) (also referred to as the aliphatic amine) to the water-insoluble carrier.

The nitrogen-containing compound is selected from the polyamine represented by Formula (1), the primary aliphatic amine represented by Formula (2), and the secondary aliphatic amine represented by Formula (3). For the nitrogen-containing compound, one kind may be used alone or a plurality of kinds may be used in combination.

In one embodiment, the nitrogen-containing compound is the polyamine represented by Formula (1).

$$R^1R^2N\text{-}X\text{-}NR^3R^4 \text{ ...} \qquad \text{Formula (1)}$$

[In Formula (1), X is a saturated or unsaturated aliphatic hydrocarbon group having 2 to 20 carbon atoms, or a heteroatom-containing carbon chain in which 1 to 5 carbon atoms of a saturated or unsaturated aliphatic hydrocarbon group having 3 to 20 carbon atoms are replaced with a nitrogen atom, a hydrogen atom that bonds to the nitrogen atom may be replaced with an alkyl group that may have an amino group, and $R^1$ to $R^4$ are each independently a hydrogen atom or an alkyl group.]

In Formula (1), X is, for example, a saturated or unsaturated aliphatic hydrocarbon group having 2 to 20 (for example, 16 or less, 14 or less, 12 or less, 10 or less, 8 or less, 6 or less, and 4 or less) carbon atoms. In Formula (1), X is, for example, a heteroatom-containing carbon chain in which 1 to 5 (for example, 1 to 3) carbon atoms of a saturated or unsaturated aliphatic hydrocarbon group having 3 to 20 (for example, 16 or less, 14 or less, 12 or less, and 10 or less) carbon atoms are replaced with a nitrogen atom. The hydrogen atom that bonds to the nitrogen atom may be replaced with an alkyl group (for example, having 1 to 6 (preferably, 1 to 4) carbon atoms) that may have an amino group. $R^1$ to $R^4$ are each independently a hydrogen atom or an alkyl group. The alkyl group, for example, has 1 to 6 (preferably, 1 to 4) carbon atoms. The aliphatic hydrocarbon group may be linear or may be branched chain.

The polyamine represented by Formula (1) is preferred to be a polyamine represented by any one of the following Formulae (1-1) to (1-6).

$$H_2N\text{-}(CH_2)_{p1}\text{-}NH_2 \text{ ...} \qquad \text{Formula (1-1)}$$

[In Formula (1-1), p1 is an integer from 2 to 12 (preferably, 2 to 6, 2 to 5, or 2 to 4), and at least one of the hydrogen atoms of the primary amino groups at both ends may be replaced with an alkyl group.],

$$H_2N\text{-}(CH_2)_{p1}\text{-}NH\text{-}(CH_2)_{p2}\text{-}NH_2 \text{ ...} \qquad \text{Formula (1-2)}$$

[In Formula (1-2), p1 and p2 are each independently an integer from 2 to 5 (preferably, 2 to 4, 2 or 3, or 2), the hydrogen atom of the secondary amino group may be replaced with an alkyl group that may have an amino group, and at least one of the hydrogen atoms of the primary amino groups at both ends may be replaced with an alkyl group.],

$$H_2N-(CH_2)_{p1}-NH-(CH_2)_{p2}-NH-(CH_2)_{p3}-NH_2 \ldots \qquad \text{Formula (1-3)}$$

[In Formula (1-3), p1, p2, and p3 are each independently an integer from 2 to 5 (preferably, 2 to 4, 2 to 3, or 2), the hydrogen atoms of the secondary amino groups may be each independently replaced with an alkyl group that may have an amino group, and at least one of the hydrogen atoms of the primary amino groups at both ends may be replaced with an alkyl group.],

$$H_2N-(CH_2)_{p1}-NH-(CH_2)_{p2}-NH-(CH_2)_{p3}-NH-(CH_2)_{p4}-NH_2 \qquad \text{Formula (1-4)}$$

[In Formula (1-4), p1, p2, p3, and p4 are each independently an integer from 2 to 5 (preferably, 2 to 4, 2 to 3, or 2), the sum of p1, p2, p3, and p4 is 17 or less, the hydrogen atoms of the secondary amino groups may be each independently replaced with an alkyl group that may have an amino group, and at least one of the hydrogen atoms of the primary amino groups at both ends may be replaced with an alkyl group.],

$$H_2N-(CH_2)_{p1}-NH-(CH_2)_{p2}-NH-(CH_2)_{p3}-NH-(CH_2)_{p4}-NH-(CH_2)_{p5}-NH_2 \, . \qquad \text{Formula (1-5)}$$

[In Formula (1-5), p1, p2, p3, p4, and p5 are each independently an integer from 2 to 5 (preferably, 2 to 4, 2 to 3, or 2), the sum of p1, p2, p3, p4 and p5 is 16 or less, the hydrogen atoms of the secondary amino groups may be each independently replaced with an alkyl group that may have an amino group, and at least one of the hydrogen atoms of the primary amino groups at both ends may be replaced with an alkyl group.],

$$H_2N-(CH_2)_{p1}-NH-(CH_2)_{p2}-NH-(CH_2)_{p3}-NH-(CH_2)_{p4}-NH-(CH_2)_{p5}-NH-(CH_2)_{p6}-NH_2 \ldots \qquad \text{Formula (1-6)}$$

[In Formula (1-6), p1, p2, p3, p4, p5, and p6 are each independently an integer from 2 to 5 (preferably, 2 to 4, 2 to 3, or 2), the sum of p1, p2, p3, p4, p5, and p6 is 15 or less, each of the hydrogen atoms of the secondary amino groups may independently be replaced with an alkyl group that may have an amino group, and at least one of the hydrogen atoms of the primary amino groups at both ends may be replaced with an alkyl group.].

In Formulae (1-2) to (1-6), the number of carbon atoms of the "alkyl group that may have an amino group" that can bond to the nitrogen atom of the secondary amino group is, for example, 1 to 6, preferably 1 to 5, preferably 1 to 4, and preferably 1 to 3. In Formulae (1-1) to (1-6), the number of carbon atoms of the "alkyl group" that can bond to the nitrogen atom of the primary amino groups at both ends is, for example, 1 to 6, preferably 1 to 5, preferably 1 to 4, and preferably 1 to 3. These "alkyl groups" are preferred to be linear or branched chain.

Examples of the polyamine represented by Formula (1) include ethylenediamine, N-ethylethylenediamine, diethylenetriamine, N-ethyldiethylenetriamine, triethylenetetramine, and tetraethylenepentamine. Besides them, the following polyamines are included: 3,3'-diaminodipropylamine; 1,3-diaminopropane; norspermidine; homospermidine; aminopropylcadaverine; aminobutylcadaverine; norspermine; thermospermine; aminopropylhomospermidine; canavalmine; homospermine; aminopentylnorspermidine; N,N-bis(aminopropyl)cadaverine; caldopentamine; homocaldopentamine; thermopentamine; caldohexamine; homocaldohexamine; thermohexamine; homothermohexamine; N[4]-aminopropylnorspermidine; N[4]-aminopropylspermidine; and N[4]-aminopropylnorspermine.

In one embodiment, the nitrogen-containing compound is the primary aliphatic amine represented by Formula (2) or the secondary aliphatic amine represented by Formula (3).

$$NH_2R^5 \ldots \qquad \text{Formula (2)}$$

[In Formula (2), $R^5$ is a saturated or unsaturated aliphatic hydrocarbon group having 1 to 12 carbon atoms.],

$$NHR^6R^7 \ldots \qquad \text{Formula (3)}$$

[In Formula (3), $R^6$ and $R^7$ are each independently a saturated or unsaturated aliphatic hydrocarbon group having 1 to 12 carbon atoms.].

In the aliphatic amine represented by Formula (2) or Formula (3), the aliphatic hydrocarbon group is preferred to be a linear or branched chain aliphatic hydrocarbon group, and is preferably a linear or branched chain saturated aliphatic hydrocarbon group. The number of carbon atoms of the aliphatic hydrocarbon group is preferably 1 to 8, preferably 1 to 6, and preferably 1 to 4.

Specific examples of the aliphatic amine include: monoalkylamines, such as ethylamine, propylamine, butylamine, pentylamine, hexylamine, heptylamine, octylamine, nonylamine, and decylamine; and dialkylamines, such as diethylamine, dipropylamine, dibutylamine, diheptylamine, dioctylamine, and dicyclohexylamine.

Examples of the nitrogen-containing compound include ethylamine, ethylenediamine, diethylamine, N-ethylethylenediamine, diethylenetriamine, N-ethyldiethylenetriamine, triethylenetetramine, or tetraethylenepentamine. Among these, ethylenediamine, diethylenetriamine, triethylenetetramine, or tetraethylenepentamine is preferred. The nitrogen-containing compound is commercially available or can be manufactured by a known method or an equivalent method thereof.

The water-insoluble carrier to which the nitrogen-containing compound binds encompasses both a water-insoluble carrier to which the above-described nitrogen-containing compound directly binds covalently and a water-insoluble carrier to which the above-described nitrogen-containing compound indirectly binds via a linker. The water-insoluble carrier to which the nitrogen-containing compound binds also encompasses one to which two or more kinds of nitrogen-containing compounds that are mutually different selected from the above-described polyamine and the above-described aliphatic amine bind.

When the polyamine represented by Formula (1) is used as the nitrogen-containing compound, a plurality of amino groups may bind to the water-insoluble carrier to form a cross-linkage structure. That is, when the polyamine represented by Formula (1) is bound to the water-insoluble carrier as the nitrogen-containing compound, at least two amino groups in the polyamine binding to the water-insoluble carrier forms the cross-linkage structure.

The nitrogen-containing compound preferably binds to the water-insoluble carrier via the amino group (or a nitrogen atom) in the compound.

When the nitrogen-containing compound binds to the water-insoluble carrier, primary amino groups, secondary amino groups, tertiary amino groups, and/or quaternary amino groups exist in the nitrogen-containing compound after binding depending on binding positions in the nitrogen-containing compound. For example, when the polyamine represented by Formula (1) binds to the water-insoluble carrier, primary amino groups, secondary amino groups, tertiary amino groups, and/or quaternary amino groups exist in the polyamine after binding depending on the binding position in the polyamine. The nitrogen-containing compound preferably binds to the water-insoluble carrier via the amino group (or the nitrogen atom) in the compound. In the Description, an "amino groups on the water-insoluble carrier" is a concept that at least includes a primary amino group, a secondary amino group, a tertiary amino group, and a quaternary amino group derived from the nitrogen-containing compound thus generated. When the nitrogen-containing compound binds via a linker, the "amino groups on the water-insoluble carrier" includes a primary amino group, a secondary amino group, a tertiary amino group, and a quaternary amino group derived from the linker. In the Description, the "total content of amino groups" means a total content ($\mu$mol) of the primary amino groups, the secondary amino groups, the tertiary amino groups, and the quaternary amino groups on the water-insoluble carrier.

The total content of amino groups on the water-insoluble carrier is not specifically limited, and is, for example, more than 0 $\mu$mol and 5,000 $\mu$mol or less per 1 g of adsorption material.

The total content of amino groups on the water-insoluble carrier can be obtained as, for example, a sum of a content of primary amino groups, a content of secondary amino groups, a content of tertiary amino groups, and a content of quaternary amino groups (quaternary ammonium groups) by measuring the amino groups using an acid-base back titration. That is, first, the adsorption material and an excessive amount of sodium hydroxide aqueous solution are added in a polypropylene container, and are sufficiently stirred at room temperature, and the amino groups to which a salt is added in the adsorption material is desalinated. Next, the adsorption material is sufficiently cleaned until the solution becomes neutral with an ion exchanged water, and is dried until the weight change becomes 1% or less. Next, the amino groups in the dried adsorption material are reacted with a constant amount of a standard solution containing excessive acid. Next, the amount of acids remained without reacting with the amino groups is titrated with the standard solution containing a base. This method ensures obtaining the total content ($\mu$mol) of amino groups. Even more specifically, the total content of amino groups on the water-insoluble carrier can be obtained by a method described in the following examples.

An amount of nitrogen-containing compound residue (an immobilizing amount of nitrogen-containing compound) can be controlled by, for example, adjusting a binding amount of the reactive functional groups to the water-insoluble carrier, a kind of nitrogen-containing compound, and a usage of the nitrogen-containing compound. The binding amount of the reactive functional groups can be controlled by, for example, reactive conditions, such as a kind of reactive functional group or a kind of solvent, an immersing temperature, or an immersing period. For example, when the water-insoluble carrier contains a polyaromatic vinyl, a binding position of the reactive functional group can also be controlled using a crosslinking agent. An amount of nitrogen-containing compound residue can be controlled by reactive conditions, such as a kind of solvent, an immersing temperature, and an immersing period, in addition to the kind of nitrogen-containing compound and the binding amount of the reactive functional groups.

The "immunosuppressive leukocyte" means a leukocyte that functions to suppress the immune system. Examples of the immunosuppressive leukocyte include a LAP positive leukocyte. The adsorption material according to the embodiment can preferably adsorb a LAP positive lymphocyte or a LAP positive monocyte among the LAP positive leukocytes, and can preferably adsorb a LAP positive CD4 positive lymphocyte, a LAP positive CD8 positive lymphocyte, or a LAP positive CD11b positive monocyte.

The "water-insoluble carrier" means a carrier that does not change its shape when it is immersed in water having a normal temperature (25°C), specifically, it is preferred to be a carrier having a weight change of 5% or less when it is immersed in the water of 25°C for one hour. Examples of the water-insoluble carrier material are not specifically limited, but preferably include polyaromatic vinyl compounds typified by polystyrenes, polyethersulfones, polysulfones, polyarylethersulfones, polyetherimides, polyimides, polyamides, and polyphenylene sulfides. The material of the water-insoluble carrier is commercially available or can be manufactured by a known method or an equivalent method thereof. These materials are materials that do not substantially have a hydroxyl group that is said to easily activate a complement when contacting blood. Among these, polystyrenes are preferred because it has many aromatic rings per unit weight and an amino group is easily immobilized to polystyrenes. These polymer materials may be used alone or may be used in combination of a plurality of kinds. The water-insoluble carrier is preferred to be a polymer material comprising a polyaromatic vinyl compound (for example, a polystyrene). The water-insoluble carrier is preferred to be a copolymer of a polystyrene and a polyolefin (for example, a copolymer of a polystyrene and a polyethylene or a copolymer of a polystyrene and a polypropylene) in that it is easy to introduce a linker, such as an active halogen group, for immobilizing an amino group to a polystyrene part and in terms of easy handling and chemical resistance due to strength reinforcement by a polyolefin part. The polymer material may be one that is blended or alloyed, and in particular, a polymer alloy of a polystyrene and a polyolefin (for example, a polymer alloy of a polystyrene and a polyethylene or a polymer alloy of a polystyrene and a polypropylene) is preferred from the standpoint that it has chemical resistance and easily retains its physical shape. Among these, the polymer alloy of a polystyrene and a polypropylene that has a proven use in a blood extracorporeal circulation therapy is preferred. It is preferred that the used water-insoluble carrier does not substantially have an amino group.

The nitrogen-containing compound may directly bind to the water-insoluble carrier or may indirectly bind to the water-insoluble carrier via a linker. Examples of a method that binds the nitrogen-containing compound to the water-insoluble carrier are not particularly limited, and includes a method that covalently binds the nitrogen-containing compound to a surface of the water-insoluble carrier via a linker by a chemical method. For example, a reactive functional group can be used as a linker. As a linker, it is preferred to be one that has an electrically neutral chemical bond, such as an amide bond, a urea bond, an ether bond, or an ester bond, and preferred to be one that has the amide bond or the urea bond. Examples of the reactive functional group as the linker can include an active halogen group, such as a halomethyl group, a haloacetyl group, a haloacetamidomethyl group, or a halogenated alkyl group, an epoxy group, a carboxyl group, an isocyanate group, a thioisocyanate group, or an acid anhydride group. Among these, the active halogen group (in particular, the haloacetyl group) is preferred because it is easily manufactured, has appropriately high reactivity, can perform an immobilizing reaction of the amino group in a mild condition, and generates a chemically stabilized covalent bind. As specific examples of the polymer to which the reactive functional group is introduced include a polystyrene to which a chloroacetamidomethyl group is added, a polysulfone to which a chloroacetamidomethyl group is added, and a polyetherimide to which a chloroacetamidomethyl group is added. It should be noted that these polymers are soluble in an organic solvent, thereby having an advantage of easy molding. While a rough indication of additive amount of nitrogen-containing compound depends on a structure of the linker, it is, for example, 10 to 10,000 $\mu$mol per 1 g of water-insoluble carrier. The nitrogen-containing compound may be used in an excessive amount.

Preliminarily reacting the reactive functional group with the water-insoluble carrier ensures the reactive functional group introduced in the water-insoluble carrier. For example, when the water-insoluble carrier is a polystyrene and the reactive functional group is a chloroacetamidomethyl group, reacting the polystyrene with N-methylol-$\alpha$-chloroacetamide ensures obtaining the polystyrene to which the chloroacetamidomethyl group is added.

The water-insoluble carrier has a form of fiber or particle. The fiber is preferably used because the fiber can have an increased area in contact with blood while securing a blood flow passage. In particular, a sea-island composite fiber is preferably used, because the sea-island composite fiber can, while retaining fiber strength with the island part, dispose a polymer material to which a ligand is easily immobilized in the sea part, spinnability and a ligand immobilizing reaction are easily ensured at the same time.

In the embodiment, the fiber or the particle that constitutes the water-insoluble carrier has a diameter of 15 to 50 $\mu$m.

In the embodiment, the reason and mechanism why the effects of the embodiment can be obtained when the diameter of the fiber or the particle that constitutes the water-insoluble carrier falls within a predetermined range are presumed as follows. When the diameter of the fiber or the particle is less than 15 $\mu$m, a packing density of the water-insoluble carrier to the column increases, and therefore, various kinds of cells, such as a platelet and the leukocyte, are easily trapped non-selectively. Among the leukocytes, since the granulocyte and the monocyte have phagocytic activities, the granulocyte and the monocyte easily detect the water-insoluble carrier itself as foreign matter when the diameter of the fiber or the particle is less than 15 $\mu$m. Meanwhile, when the diameter of the fiber or the particle is greater than 50 $\mu$m, since the blood contacted area of the water-insoluble carrier per unit volume decreases, an adsorption rate of the LAP positive leukocyte lowers. When filling in the adsorption column, it is possible to increase the adsorption rate of LAP positive cell if an amount of the filled carrier is increased, however, this increases a capacity of the column, which leads to increase of a blood amount that needs to be taken out of a body. Accordingly, it is presumed that the diameter of the fiber or the particle constituting the water-insoluble carrier is required to be 15 to 50 $\mu$m. It should be noted that these presumptions do not limit the embodiment. The diameter of the fiber or the particle is preferably 17 $\mu$m or more, 20 $\mu$m or more, and 25 $\mu$m or more. The diameter of the fiber or the particle is preferably 40 $\mu$m or less, 35 $\mu$m or less, and 30 $\mu$m or less. Any of the preferred lower limit values can be combined with any of the preferred upper limit values.

The "diameter of fiber" can be obtained by the following method. First, a hundred samples of fibers are randomly extracted, and one photograph of a cross-sectional surface (a cross-sectional surface perpendicular to the extension direction of the fiber) is taken for each one sample at 1,000 to 3,000-fold magnification using a scanning electron microscope. Next, the diameters of the respective fiber cross-sectional surfaces are measured. Calculating a mean value of those values (a mean value of the diameters of total of a hundred cross-sectional surfaces) obtains the "diameter of fiber." When the fiber cross-sectional surface is not a circle, a diameter of a circle that has the same area as the cross-sectional area is the diameter of fiber.

The "diameter of particle" can be obtained by the following method. First, ten sample groups of particles are randomly extracted, and one photograph is taken for each one sample group at 1000 to 3000-fold magnification using a scanning electron microscope. Next, diameters of ten particles per one photograph are measured. Calculating a mean value of those values (a mean value of the diameters of total of a hundred particles) obtains the "diameter of particle." When a pictured shape of the particle is not a circle, a diameter of a circle that has the same area as the particle area is the diameter of particle.

In the embodiment, the water-insoluble carrier has a surface with an arithmetic mean roughness of 0.1 to 3.0 $\mu$m.

The "arithmetic mean roughness" means a mean value of absolute values of deviations from an average line to a measurement curved line of an extracted part that is extracted by a reference length L in the direction of the average line from a roughness surface, and means an arithmetic mean roughness (Ra) in Japanese Industrial Standard B 0601-2001. The arithmetic mean roughness can be measured with, for example, a shape measurement laser microscope. For the measurement environment, it is preferred that the measurement be performed with the water-insoluble carrier being wet with water. In the case where there is an orientation as with a fiber, a value in the longitudinal direction is measured.

In the embodiment, the reason and mechanism why the effects of the embodiment can be obtained when the arithmetic mean roughness of the surface of the water-insoluble carrier falls within a predetermined range are presumed as follows. When the arithmetic mean roughness of the surface of the water-insoluble carrier is greater than 3.0 $\mu$m, the granulocyte and the monocyte having the phagocytic activities easily detect unevenness of the water-insoluble carrier as foreign matter. Therefore, the granulocyte and the monocyte are easily adsorbed to the surface, thereby lowering selectivity of the LAP positive leukocyte. Meanwhile, when the arithmetic mean roughness of the surface of the water-insoluble carrier is smaller than 0.1 $\mu$m, the adsorption rate of the LAP positive leukocyte lowers. This is presumably because the increased contacted area between the water-insoluble carrier and blood causes the blood components other than the LAP positive leukocyte to be easily in contact and causes the LAP positive leukocyte to have a difficulty in efficiently being in contact with the surface of the water-insoluble carrier. It should be noted that these presumptions do not limit the embodiment. The arithmetic mean roughness of the surface of the water-insoluble carrier is preferably 0.5 $\mu$m or more, 0.7 $\mu$m or more, 0.9 $\mu$m or more, and 1.0 $\mu$m or more. The arithmetic mean roughness of the surface of the water-insoluble carrier is preferably 2.0 $\mu$m or less, 1.8 $\mu$m or less, and 1.5 $\mu$m or less. Any of the preferred lower limit values can be combined with any of the preferred upper limit values. It should be noted that the preferred diameter of the above-described fiber or the above-described particle and the preferred arithmetic mean roughness of the surface of the water-insoluble carrier can be conveniently combined.

When the arithmetic mean roughness of the surface of the water-insoluble carrier is 0.1 to 3.0 $\mu$m, it is possible to suppress an adhesion of the platelets. That is, when the nitrogen-containing compound is bound to the water-insoluble carrier, the platelets tend to easily adhere, but when the arithmetic mean roughness of the surface of the water-insoluble carrier is 0.1 to 3.0 $\mu$m, it is possible to suppress the tendency of the platelet adhesion.

The arithmetic mean roughness of the surface of the water-insoluble carrier can be, for example, controlled by immersing the water-insoluble carrier in an organic solvent. Examples of methods for controlling the arithmetic mean roughness of the surface of the water-insoluble carrier include a method that a polymer obtained by mixing a polyaromatic vinyl compound and a polypropylene as a water-insoluble carrier is immersed in a solvent that can partly dissolve the polyaromatic vinyl compound and that does not dissolve the polypropylene. The arithmetic mean roughness of the surface of the water-insoluble carrier can be controlled by, for example, a kind of polymer, a molecular weight of polymer, a kind of solvent, an immersing temperature, and an immersing period. Furthermore, for the polyaromatic vinyl, introducing a crosslinking agent ensures employing a method that, for example, controls the solubility to a solvent. Furthermore, the above-described reaction can also be simultaneously performed with an introduction reaction of the nitrogen-containing compound.

The fiber is preferred in that the fiber can have an increased area in contact with blood while securing a blood flow passage by high-order processing. Among them, a sea-island type composite fiber is preferred, and from a standpoint of retaining strength as a material, a sea-island type composite fiber whose island is a reinforcing material and sea is an alloy of a water-insoluble polymer and a reinforcing material is preferred, and furthermore, a sea-island type composite fiber in which island is polypropylene and sea is an alloy of polystyrene and polypropylene is preferred. Examples of the reinforcing material are not specifically limited, and include polyamides, polyacrylonitriles, polyethylenes, polypropylenes, nylons, polymethyl methacrylates, and polytetrafluoroethylenes. Among these, polypropylenes are preferred. These polymers may be used alone or may be used in combination of a plurality of kinds.

When the form of the water-insoluble carrier is the fiber, it is preferred to be a knitted fabric as its high-order processed product. The knitted fabric can secure a blood flow passage by controlling its stitch, thereby ensuring reduced pressure loss when blood passes through the fiber. When the knitted fabric is formed by doubling the fibers, the number of doublings is preferably 10 to 100, and preferably 30 to 80. When the number of doublings is 100 or less, the LAP positive leukocyte easily flows into a fiber bundle, thereby ensuring an improved adsorption rate. When the number of doublings is 10 or more, maintainability of the form of the knitted fabric improves. It should be noted that any of the preferred lower limit values can be combined with any of the preferred upper limit values.

The adsorption material of the embodiment can be used for an adsorption carrier for the immunosuppressive leukocyte (in particular, the LAP positive leukocyte), and can be used as a filler of the adsorption column.

The adsorption material of the embodiment has both adsorption rates of LAP positive lymphocyte and monocyte of preferably 30% or more, preferably 35% or more, and preferably 40% or more. As for selectivity, both the lymphocyte and the monocyte have the adsorption rates of LAP positive lymphocyte and monocyte of preferably 2.5 times or more, preferably 5 times or more, and preferably 8 times or more of respective adsorption rates of the LAP negative lymphocyte and monocyte. Here, a CD4-positive leukocyte or a CD8-positive leukocyte are subjects as the lymphocytes, and it is only necessary that any of the CD4-positive leukocyte or the CD8-positive leukocyte satisfies the above-described selectivity, and it is preferred that both the CD4-positive leukocyte and the CD8-positive leukocyte satisfy the above-described selectivity. A CD11b-positive leukocyte is a subject as the monocyte. Examples of a test system of the above-described adsorption rate include a batch leukocyte adsorption test (for example, see the embodiment) using human blood. Examples of an evaluation system include an analysis (for example, see the embodiment) by a flow cytometry using a surface antigen of the leukocyte as an index. Furthermore, the adhesion of the platelets has possibilities to be a cause of the reduced adsorption rates of the LAP positive lymphocyte and monocyte and also to cause clogging of the column, and therefore, the adhesion of the platelets is desired to be as less as possible. Accordingly, the adsorption rate of the platelets is preferably 80% or less, preferably 70% or less, and preferably 65% or less. The adsorption rate of the platelets can be evaluated by a batch test and a blood cell counter similarly to the above (for example, see the embodiment).

The adsorption column of the embodiment includes the adsorption material of the embodiment.

The "adsorption column" means one that has at least a blood inlet portion, a housing portion, and a blood outlet portion, and the housing portion is filled with the adsorption material. Exemplary adsorption columns include a radial flow type adsorption column. As described above, the form of the adsorption material is preferably a fiber, and preferably a knitted fabric.

An exemplary configuration of an inside of the adsorption column will be described along FIG. 1. In FIG. 1, reference numeral 1 denotes a container body, and there are an inflow port 2 and an outflow port 3 at a front end and a rear end in its longitudinal direction. The inflow port 2 has an inside where a filter 4 and a circular plate-shaped partition plate 5 are disposed. The outflow port 3 has an inside where a filter 6 and a circular plate-shaped partition plate 7 are disposed. Among the two partition plates 5 and 7, the partition plate 5 in the front side (inflow port side) has an opening 5a in the center, and the partition plate 7 in the rear side has a center portion where a support protrusion 7a is disposed. The partition plate 7 has an outer periphery where multiple through holes 7b are intermittently provided in the circumferential direction. Furthermore, one pipe 8 is bridged between the opening 5a of the partition plate 5 and the support protrusion 7a of the partition plate 7. The pipe 8 internally has a flow passage 9 that introduces blood and has a peripheral wall on which multiple through-holes 10 are provided. The pipe 8 has its front end communicating with the opening 5a of the partition plate 5 and has its rear end closed with the support protrusion 7a of the partition plate 7. The outer periphery of this pipe 8 is wound with a plurality of layers of an adsorption material 11 for many times. When this adsorption column is used for a circulation method, tubes are coupled to the inflow port 2 and the outflow port 3 that forms a circulation circuit with a blood pool. The blood taken out of the blood pool is supplied to the inflow port 2, a target adsorption substance (the immunosuppressive leukocyte) is removed with the adsorption material 11 inside, and the blood is flown out of the outflow port 3 to circulate the blood so as to return to the blood pool again. In the column, the blood that entered the flow passage 9 through the filter 4 from the inflow port 2 moves through the flow passage 9 and sequentially infiltrates into the adsorption material 11 from the through-holes 10 to move to any of the radial directions while cells and the like are adsorbed. The blood from which the cells and the like are removed flows out of the multiple through holes 7b on the outer periphery of the partition plate 7, and flows out of the outflow port 3 through the filter 6. While in the above-described example, the blood flows out of the through-holes 10 while flowing through the flow passage 9 inside the pipe 8 from the opening 5a, the moving direction of the blood in the adsorption column may be inverted from the above to supply the blood from the outflow port 3 and be flown out of the inflow port 2.

In order to increase the adsorption rate of the LAP positive leukocyte, a blood linear velocity in the column is also important. That is, when the blood linear velocity is fast, there may be a case where a sufficient interaction of the LAP positive leukocyte with the adsorption material becomes difficult to happen. Meanwhile, when the blood linear velocity is slow, there may be a case where other blood components, such as a platelet and a protein, non-specifically adhere to the adsorption material to inhibit the interaction between the adsorption material and the LAP positive leukocyte. Accordingly, the maximum value of blood linear velocity in the adsorption material when a flow rate Sin of the adsorption column inlet is 50 $cm^3$/minute is preferably 50 cm/minute or less, and preferably 25 cm/minute or less. The minimum value of blood linear velocity in the adsorption material when the flow rate of the adsorption column inlet is 50 $cm^3$/minute is preferably 0.1 cm/minute or more and preferably 0.5 cm/minute or more. Here, the blood linear velocity is obtained by a calculation, and, for example, in the case of the following radial flow type adsorption column, the maximum value ($V_{max}$) of the blood linear velocity in the adsorption material is calculated from a total area ($S_p$) of the openings that open on the side surface of the center pipe and the flow rate Sin (50 $cm^3$/minute) of the adsorption column inlet by the following Formula 1.

$$V_{max}(cm/minute) = S_{in}(cm^3/minute)/S_p(cm^2) \ ... \ \text{Formula 1}$$

The minimum value ($V_{min}$) is calculated from an area ($S_o$) of an outermost peripheral surface of the adsorption material wound around the center pipe and the flow rate Sin (50 $cm^3$/minute) of the adsorption column inlet by the following Formula 2.

$$V_{min}(cm/minute) = S_{in}(cm^3/minute)/S_o(cm^2) \ ... \ \text{Formula 2}$$

Meanwhile, when the form of the adsorption material is a fiber form made of particles or made by simply stacking fibers, the above-described maximum value and the above-described minimum value are the same values.

Furthermore, the adsorption column is preferred to be a radial flow type adsorption column that includes a center pipe, a plate A, and a plate B. The center pipe has a side surface in the longitudinal direction where through-holes are provided for flowing out the supplied blood. The adsorption material is filled around the above-described center pipe. The plate A is communicated through the upstream end of the above-described center pipe for causing the above-described blood that flows in to pass inside the above-described center pipe and is disposed to prevent the above-described blood from contacting the adsorption material without passing through the center pipe. The plate B is disposed to close the downstream end of the above-described center pipe and secure the adsorption material in a space around the above-described center pipe. This causes the blood to uniformly flow through the adsorption material. It should be noted that when an aperture ratio of the through-holes of the above-described center pipe is low, the pressure loss easily occurs in this part, and therefore, granulocytes, monocytes, and platelets are activated so that they easily adhere to the adsorption material. Therefore, there may be a case where the adsorption selectivity of the LAP positive leukocyte lowers. When the aperture ratio is high, it is possible to have problems, such as reduced strength of the pipe and easy occurrence of a short path at the through-holes near the blood inlet portion. Accordingly, the aperture ratio of the through-holes is preferably 20 to 80%, and preferably 30 to 60%.

The "radial flow type" means the way the blood flows inside the column. When the blood is flown in the perpendicular direction to the inlet and the outlet of the column, and there is the blood flow in the horizontal direction inside the column, it is referred to as a radial flow type.

The "aperture ratio of through-holes" means a value obtained by the following Formula 3.

Aperture ratio of through-holes (%) = sum of areas of through-holes formed on side surface in longitudinal direction of pipe/area of side surface of pipe × 100 ... Formula 3

The adsorption column of the embodiment can be used in a blood purification therapy. Using the adsorption column of the embodiment as a column for blood purification ensures selectively removing the immunosuppressive leukocyte from blood. For example, extracorporeally circulating blood and passing the blood through the adsorption column of the embodiment ensures selectively removing the immunosuppressive leukocyte from the blood. That is, the adsorption column of the embodiment can be used as a column for extracorporeal circulation. More specifically, the adsorption column of the embodiment can be used for a therapy that selectively removes the immunosuppressive leukocyte from blood of a cancer patient. That is, the adsorption column of the embodiment can be used as a column for cancer therapy.

The adsorption column of the embodiment is appropriately used for cancer therapy since it can selectively remove the immunosuppressive leukocyte. It is also possible to use in combination with a cell infusion treatment that activates dendritic cells, natural killer cells, and the like.

While the following describes the embodiment with examples, the embodiment is not limited to these examples.

Examples

1. Measuring Arithmetic Mean Roughness of Surface

In the examples, an arithmetic mean roughness of a surface of the water-insoluble carrier included in the adsorption material was measured by the following method.

Using a shape measuring laser microscope (made by KEYENCE CORPORATION; Color 3D Laser Scanning Microscope VK-9700), a surface of the adsorption material in a state of being wet with water so as not to be dried was observed at 100-fold magnification, and an arithmetic mean roughness of the surface was measured (compliant with Japanese Industrial Standard B 0601-2001). A reference length L was 50 $\mu$m, and a mean value of absolute values of deviations measured at ten different positions was the value of the arithmetic mean roughness of the surface. It should be noted that as the state of being wet with water is preferably a state where a moisture percentage (moisture percentage = 100 $\times$ moisture weight/material weight) is 20% or more.

2. Adsorption Test of Immunosuppressive Leucocyte

In the example, an adsorption rate of the immunosuppressive leukocytes of the adsorption material was measured by a batch adsorption test using human blood. A flow cytometry (FACSCalibur, made by Becton, Dickinson and Company) was used for an analysis.

First, five adsorption materials cut out into disk shapes with diameters of 10 mm were put into a polypropylene container. 3.07 mL of blood drawn from a healthy human was added to this container, and was mixed by inverting for one hour in an incubator at 37°C. After the adsorption materials were removed from the container, the blood remained in the container was put into a separation tube (made by Greiner Bio-One International GmbH, LeucoSep™) with a capacity of 15 mL and was centrifuged. The separated cell layer (the leukocyte) was collected, resuspended in PBS (-), and centrifuged, and the cell layer (the leukocyte) was collected. The collected number of leukocytes and number of LAP positive leukocytes were calculated using the flow cytometry. The number of LAP negative leukocytes is the number of leukocytes that do not bind to the LAP and was calculated by subtracting the number of LAP positive leukocytes from the number of leukocytes. The number of leukocytes is a sum of the number of LAP positive leukocytes and the number of LAP negative leukocytes.

The analysis of the surface antigen of the leukocyte was performed using the flow cytometry (FACSCalibur made by Becton, Dickinson and Company). As cell surface staining antibodies, Phycoerythrin (PE) anti-human LAP by R&D Systems, Inc., FITC anti-human CD4 Antibody, APC anti-human CD8 Antibody, and APC anti-human CD11b Antibody by BioLegend, Inc. were used. For the LAP positive lymphocyte, a LAP positive CD4 positive lymphocyte and a LAP positive CD8 positive lymphocyte were evaluation targets, and for the LAP positive monocyte, a LAP positive CD11b positive monocyte was an evaluation target.

The adsorption rate was calculated by the following Formula.

Adsorption rate (%) = (number of LAP positive leukocytes in blood without addition of adsorption material − number of LAP positive leukocytes in blood with addition of adsorption material) / (number of LAP positive leukocytes in blood without addition of adsorption material $\times$ 100

3. Adhesion Test of Platelet

With the same experiment system as used in the adsorption test of the immunosuppressive leukocyte, an adhesion rate of the platelets was calculated. The number of platelets was a measured with a blood cell counter.

The adhesion rate of the platelets was calculated by the following Formula.

$$\text{Adhesion rate (\%) of platelets} = (\text{number of platelets in blood without addition of adsorption material} - \text{number of platelets in blood with addition of adsorption material}) / \text{number of platelets in blood without addition of adsorption material} \times 100$$

4. Measuring Diameter of Fiber of Water-Insoluble Carrier

A "diameter of fiber" was obtained by the following method. First, a hundred samples of fibers were randomly extracted, and one photograph of a cross-sectional surface (a cross-sectional surface perpendicular to the extension direction of the fiber) was taken for each one sample at 3000-fold magnification using a scanning electron microscope. Next, the diameters of the respective fiber cross-sectional surfaces were measured. Calculating a mean value of those values (a mean value of the diameters of total of a hundred cross-sectional surfaces) obtained the "diameter of fiber." When the fiber cross-sectional surface was not a circle, a diameter of a circle that had the same area as the cross-sectional area was the diameter of fiber.

5. Manufacturing Raw Knitted Fabric (Water-Insoluble Carrier) and Intermediate (Ligand Bound Water-Insoluble Carrier)

(1) Raw Knitted Fabric 1 and Intermediate 1

As a water-insoluble carrier, a sea-island composite fiber (diameter of fiber: 20 μm) was spun. The sea-island composite fiber had 16 islands of island component made of polypropylene (Prime Polymer Co., Ltd.; J105WT) and a sea component made of 90 weight% of polystyrene (weight average molecular weight: 181,000) and 10 weight% of polypropylene (Prime Polymer Co., Ltd.; J105WT), while a ratio of the island and the sea (weight ratio) was 50:50. The obtained 42 fibers were combined to form a knitted fabric (hereinafter, a raw knitted fabric 1). It should be noted that a roughness of a fiber surface is affected by the number of islands, a sea/island ratio, molecular weights of polystyrene and polypropylene, and the like.

Paraformaldehyde (hereinafter, PFA) (0.26 g) was dissolved in a mixed solution of nitrobenzene (50 mL) and sulfuric acid (32 mL) at 10°C (hereinafter, a PFA solution). Furthermore, N-methylol-α-chloroacetamide (18 g) was dissolved in a mixed solution of nitrobenzene (50 mL) and sulfuric acid (32 mL) at 10°C (hereinafter, an NMCA solution). After immersing the raw knitted fabric 1 (10 g) in the PFA solution, the NMCA solution was promptly added and stirred. After immersion and stirring for one hour, the knitted fabric was taken out. After cleaning with excessive nitrobenzene, the knitted fabric was displaced and cleaned with methanol, and further cleaned with water, to obtain an α-chloracetamidomethylated knitted fabric (hereinafter, an intermediate 1). A series of operation from the manufacturing of the PFA solution to the cleaning of the knitted fabric using the methanol was performed at 15°C or less.

(2) Raw Knitted Fabric 2 and Intermediate 2

As a water-insoluble carrier, a sheath-core composite fiber (diameter of fiber: 5 μm) was spun. The sheath-core composite fiber had a core component made of polypropylene (Prime Polymer Co., Ltd.; J105WT) and a sheath component made of 90 weight% of polystyrene (weight average molecular weight: 261,000) and 10 weight% of polypropylene (Prime Polymer Co., Ltd.; J105WT), while a ratio of the core and the sheath (weight ratio) was 50:50. The obtained 42 fibers were combined to form a knitted fabric (hereinafter, a raw knitted fabric 2).

Except that the raw knitted fabric 2 was used instead of the raw knitted fabric 1, the process similar to that described above was performed to obtain an α-chloracetamidomethylated knitted fabric (hereinafter, an intermediate 2).

(3) Raw Knitted Fabric 3 and Intermediate 3

As a water-insoluble carrier, a sea-island composite fiber (diameter of fiber: 20 μm) was spun. The sea-island composite fiber had 16 islands of island component made of polypropylene (Prime Polymer Co., Ltd.; J105WT) and a sea component made of polystyrene (weight average molecular weight: 261,000), while a ratio of the island and the sea (weight ratio) was 30:70. The obtained 42 fibers were combined to form a knitted fabric (hereinafter, a raw knitted fabric 3).

Except that the raw knitted fabric 3 was used instead of the raw knitted fabric 1, the process similar to that described above was performed to obtain an α-chloracetamidomethylated knitted fabric (hereinafter, an intermediate 3).

(4) Raw Knitted Fabric 4 and Intermediate 4

As a water-insoluble carrier, a sheath-core composite fiber (diameter of fiber: 10 μm) was spun. The sheath-core composite fiber had a core component made of polypropylene (Prime Polymer Co., Ltd.; J105WT) and a sheath component made of 90 weight% of polystyrene (weight average molecular weight: 261,000) and 10 weight% of polypropylene (Prime Polymer Co., Ltd.; J105WT), while a ratio of the core and the sheath (weight ratio) was 50:50. The obtained 42 fibers were combined to form a knitted fabric (hereinafter, a raw knitted fabric 4).

Except that the raw knitted fabric 4 was used instead of the raw knitted fabric 1, the process similar to that described above was performed to obtain an α-chloracetamidomethylated knitted fabric (hereinafter, an intermediate 4).

(5) Raw Knitted Fabric 5 and Intermediate 5

As a water-insoluble carrier, a sea-island composite fiber (diameter of fiber: 40 μm) was spun. The sea-island composite fiber had 16 islands of island component made of polypropylene (Prime Polymer Co., Ltd.; J105WT) and a sea component made of polystyrene (weight average molecular weight: 261,000), while a ratio of the island and the sea (weight ratio) was 50:50. The obtained 42 fibers were combined to form a knitted fabric (hereinafter, a raw knitted fabric 5).

Except that the raw knitted fabric 5 was used instead of the raw knitted fabric 1, the process similar to that described above was performed to obtain an α-chloracetamidomethylated knitted fabric (hereinafter, an intermediate 5).

(6) Raw Knitted Fabric 6 and Intermediate 6

As a water-insoluble carrier, a sea-island composite fiber (diameter of fiber: 50 μm) was spun. The sea-island composite fiber had 16 islands of island component made of polypropylene (Prime Polymer Co., Ltd.; J105WT) and a sea component made of polystyrene (weight average molecular weight: 261,000), while a ratio of the island and the sea (weight ratio) was 50:50. The obtained 42 fibers were combined to form a knitted fabric (hereinafter, a raw knitted fabric 6).

Except that the raw knitted fabric 6 was used instead of the raw knitted fabric 1, the process similar to that described above was performed to obtain an α-chloracetamidomethylated knitted fabric (hereinafter, an intermediate 6).

6. Manufacturing Adsorption Material

(Example 1)

The intermediate 1 (10 g) was immersed in a solution in which diethylenetriamine (hereinafter, DETA) (929 μL) and triethylamine (28.6 mL) were dissolved in dimethylsulfoxide (398 mL) under stirring for one hour at room temperature. Afterwards, the intermediate 1 processed with the diethylenetriamine was cleaned with water and dried, to obtain an adsorption material E1.

(Example 2)

The intermediate 1 (10 g) was immersed in a solution in which tetraethylenepentamine (hereinafter, TEPA) (1640 μL) and triethylamine (28.6 mL) were dissolved in dimethylsulfoxide (398 mL) under stirring for one hour at room temperature. Afterwards, the intermediate 1 processed with the tetraethylenepentamine was cleaned with water and dried, to obtain an adsorption material E2.

(Comparative Example 1)

The raw knitted fabric 1 was used as an adsorption material C1.

(Comparative Example 2)

Except that the intermediate 2 was used instead of the intermediate 1, the process similar to that described in Example 1 was performed to obtain an adsorption material C2.

(Comparative Example 3)

The intermediate 3 (10 g) was dipped in a solution in which diethylenetriamine (DETA) (929 μL) and triethylamine (28.6 mL) were dissolved in dimethylsulfoxide (398 mL), and was warmed to 80°C and immersed under stirring for ten hours. Afterwards, the knitted fabric processed with the diethylenetriamine was cleaned with water and dried, to obtain an adsorption material C3.

(Example 3)

Except that an immersing period was ten minutes, the process similar to that described in Example 1 was performed to obtain an adsorption material E3.

(Example 4)

Except that an immersing period was two hours, the process similar to that described in Comparative Example 3 was performed to obtain an adsorption material E4.

(Comparative Example 4)

Except that the intermediate 4 was used instead of the intermediate 1, the process similar to that described in Example 1 was performed to obtain an adsorption material C4.

(Example 5)

Except that the intermediate 5 was used instead of the intermediate 1, the process similar to that described in Example 1 was performed to obtain an adsorption material E5.

(Example 6)

Except that the intermediate 6 was used instead of the intermediate 1, the process similar to that described in Example 1 was performed to obtain an adsorption material E6.

(Comparative Example 5)

Except that an immersing period was three minutes, the process similar to that described in Example 1 was performed to obtain an adsorption material C5.

(Example 7)

Except that an immersing period was one hour, the process similar to that described in Comparative Example 3 was performed to obtain an adsorption material E7.
Arithmetic mean roughnesses of surfaces and adsorption rates of the immunosuppressive leukocytes, and attachment rates of the platelets were measured for the adsorption materials (E1 to E7) and the adsorption materials (C1 to C5) by the above-described methods. The results are illustrated in Tables 1-1 to 1-3.

[Table 1-1]

| | | Example 1 | Example 2 | Comparative Example 1 | Comparative Example 2 | Comparative Example 3 |
|---|---|---|---|---|---|---|
| Adsorption Material | | E1 | E2 | C1 | C2 | C3 |
| Water-insoluble Carrier | Fiber Diameter ($\mu$m) | 20 | 20 | 20 | 5 | 20 |
| | Arithmetic Mean Roughness ($\mu$m) | 1.1 | 1.2 | 0.0 | 0.7 | 4.8 |
| | Nitrogen-Containing Compound | DETA | TEPA | None | DETA | DETA |
| Lymph Corpuscle/CD4 (+) | LAP (+) Cell Adsorption Rate (%) | 66 | 60 | 25 | 64 | 63 |
| | LAP (-) Cell Adsorption Rate (%) | 2 | 4 | 23 | 62 | 65 |
| | LAP (+) Cell Adsorption Rate (%)/LAP (-) Cell Adsorption Rate (%) | 33 | 15 | 1.1 | 1.0 | 1.0 |
| Lymph Corpuscle/CD8 (+) | LAP (+) Cell Adsorption Rate (%) | 43 | 42 | 1 | 33 | 30 |
| | LAP (-) Cell Adsorption Rate (%) | 1 | 5 | 2 | 35 | 29 |
| | LAP (+) Cell Adsorption Rate (%)/LAP (-) Cell Adsorption Rate (%) | 43 | 8.4 | 0.5 | 0.9 | 1.0 |
| Mononuclear Leucocyte/CD11b (+) | LAP (+) Cell Adsorption Rate (%) | 65 | 63 | 25 | 60 | 58 |
| | LAP (-) Cell Adsorption Rate (%) | 2 | 3 | 13 | 59 | 58 |
| | LAP (+) Cell Adsorption Rate (%)/LAP (-) Cell Adsorption Rate (%) | 33 | 21 | 1.9 | 1.0 | 1.0 |
| Adhesion Rate of Platelets (%) | | 45 | 65 | 35 | 93 | 87 |

[Table 1-2]

| | | Example 3 | Example 4 | Comparative Example 4 | Example 5 | Example 6 |
|---|---|---|---|---|---|---|
| Adsorption Material | | E3 | E4 | C4 | E5 | E6 |
| Water-insoluble Carrier | Fiber Diameter ($\mu$m) | 20 | 20 | 10 | 40 | 50 |
| | Arithmetic Mean Roughness ($\mu$m) | 0.2 | 2.7 | 1.2 | 1.5 | 1.3 |
| | Nitrogen-Containing Compound | DETA | DETA | DETA | DETA | DETA |
| Lymph Corpuscle/CD4 (+) | LAP (+) Cell Adsorption Rate (%) | 49 | 71 | 79 | 51 | 30 |
| | LAP (-) Cell Adsorption Rate (%) | 2 | 24 | 40 | 3 | 2 |
| | LAP (+) Cell Adsorption Rate (%)/LAP (-) Cell Adsorption Rate (%) | 25 | 3.0 | 2.0 | 17 | 15 |
| Lymph Corpuscle/CD8 (+) | LAP (+) Cell Adsorption Rate (%) | 42 | 61 | 75 | 49 | 26 |
| | LAP (-) Cell Adsorption Rate (%) | 4 | 21 | 45 | 5 | 2 |
| | LAP (+) Cell Adsorption Rate (%)/LAP (-) Cell Adsorption Rate (%) | 11 | 2.9 | 1.7 | 9.8 | 13 |
| Mononuclear Leucocyte/CD11b (+) | LAP (+) Cell Adsorption Rate (%) | 50 | 65 | 80 | 43 | 39 |
| | LAP (-) Cell Adsorption Rate (%) | 3 | 17 | 71 | 2 | 4 |
| | LAP (+) Cell Adsorption Rate (%)/LAP (-) Cell Adsorption Rate (%) | 17 | 3.8 | 1.1 | 22 | 9.8 |
| Adhesion Rate of Platelets (%) | | 54 | 62 | 88 | 47 | 39 |

[Table 1-3]

| | | Comparative Example 5 | Example 7 |
|---|---|---|---|
| Adsorption Material | | C5 | E7 |
| Water-insoluble Carrier | Fiber Diameter ($\mu$m) | 20 | 20 |
| | Arithmetic Mean Roughness ($\mu$m) | 0.0 | 2.1 |
| | Nitrogen-Containing Compound | DETA | DETA |
| Lymph Corpuscle/CD4 (+) | LAP (+) Cell Adsorption Rate (%) | 26 | 69 |
| | LAP (-) Cell Adsorption Rate (%) | 10 | 10 |
| | LAP (+) Cell Adsorption Rate (%)/LAP (-) Cell Adsorption Rate (%) | 2.6 | 6.9 |

(continued)

|  |  | Comparative Example 5 | Example 7 |
|---|---|---|---|
| Adsorption Material | | C5 | E7 |
| Lymph Corpuscle/CD8 (+) | LAP (+) Cell Adsorption Rate (%) | 16 | 65 |
| | LAP (-) Cell Adsorption Rate (%) | 3 | 12 |
| | LAP (+) Cell Adsorption Rate (%)/LAP (-) Cell Adsorption Rate (%) | 5.3 | 5.4 |
| Mononuclear Leucocyte/CD11b (+) | LAP (+) Cell Adsorption Rate (%) | 22 | 73 |
| | LAP (-) Cell Adsorption Rate (%) | 8 | 11 |
| | LAP (+) Cell Adsorption Rate (%)/LAP (-) Cell Adsorption Rate (%) | 2.8 | 6.6 |
| Adhesion Rate of Platelets (%) | | 40 | 55 |

Abbreviations in the table are as follows.
CD4 (+): CD4 positive
CD8 (+): CD8 positive
CD11b (+): CD11 positive
LAP (+): Latency Associated Peptide positive
LAP (-): Latency Associated Peptide negative
DETA: diethylenetriamine
TEPA: tetraethylenepentamine

Industrial Applicability

The adsorption material and the adsorption column of the embodiment can adsorb the immunosuppressive leukocyte. Therefore, an application to cancer therapy is expected. The adsorption material and the adsorption column of the embodiment can be used in combination with the cell infusion treatment that activates the dendritic cell, the natural killer cell, and the like.

Reference Signs List

1    Container body
2    Inflow port
3    Outflow port
4    Filter
5    Partition plate
5a    Opening of partition plate
6    Filter
7    Partition plate
7a    Support protrusion of partition plate
7b    Through hole of partition plate
8    Pipe
9    Flow passage
10    Through-hole
11    Adsorption material
Q    Blood flow

**Claims**

1. An adsorption material for immunosuppressive leukocyte, the adsorption material comprising a water-insoluble carrier to which at least one nitrogen-containing compound is bound, the nitrogen-containing compound being selected from a polyamine represented by following Formula (1), a primary aliphatic amine represented by following Formula (2), and a secondary aliphatic amine represented by Formula (3), wherein the water-insoluble carrier has a form of fiber or particle, the fiber or the particle has a diameter of 15 to 50 $\mu$m, and the water-insoluble carrier has a surface with an arithmetic mean roughness of 0.1 to 3.0 $\mu$m:

$$R^1R^2N\text{-}X\text{-}NR^3R^4 \ldots \qquad \text{Formula (1)}$$

in Formula (1), X is a saturated or unsaturated aliphatic hydrocarbon group having 2 to 20 carbon atoms, or a heteroatom-containing carbon chain in which 1 to 5 carbon atoms of a saturated or unsaturated aliphatic hydrocarbon group having 3 to 20 carbon atoms are replaced with a nitrogen atom, a hydrogen atom that bonds to the nitrogen atom may be replaced with an alkyl group that may have an amino group, and $R^1$ to $R^4$ are each independently a hydrogen atom or an alkyl group;

$$NH_2R^5 \ldots \qquad \text{Formula (2)}$$

in Formula (2), $R^5$ is a saturated or unsaturated aliphatic hydrocarbon group having 1 to 12 carbon atoms;

$$NHR^6R^7 \ldots \qquad \text{Formula (3)}$$

in Formula (3), $R^6$ and $R^7$ are each independently a saturated or unsaturated aliphatic hydrocarbon group having 1 to 12 carbon atoms.

2. The adsorption material according to claim 1, wherein the nitrogen-containing compound comprises the polyamine represented by the Formula (1).

3. The adsorption material according to claim 1 or 2, wherein the nitrogen-containing compound binds to the water-insoluble carrier via a linker.

4. The adsorption material according to any one of claims 1 to 3, wherein the water-insoluble carrier has a form of fiber.

5. The adsorption material according to any one of claims 1 to 4, wherein the immunosuppressive leukocyte is a LAP positive lymphocyte or a LAP positive monocyte.

6. An adsorption column comprising the adsorption material according to any one of claims 1 to 5.

7. The adsorption column according to claim 6, wherein the adsorption column is used for a blood purification therapy.

Fig. 1

## INTERNATIONAL SEARCH REPORT

| | International application No. |
|---|---|
| | PCT/JP2018/033129 |

### A. CLASSIFICATION OF SUBJECT MATTER
Int.Cl. A61M1/36(2006.01)i, B01J20/26(2006.01)n, B01J20/28(2006.01)n

According to International Patent Classification (IPC) or to both national classification and IPC

### B. FIELDS SEARCHED

Minimum documentation searched (classification system followed by classification symbols)
Int.Cl. A61M1/02-1/36, B01J20/26, B01J20/28

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2012/133399 A1 (SHIGA UNIVERSITY OF MEDICAL SCIENCE) 04 October 2012, entire text, all drawings & US 9127250 B2 | 1-7 |
| A | JP 2006-288571 A (TORAY INDUSTRIES, INC.) 26 October 2006, entire text, all drawings & US 2009/0275874 A1 & WO 2006/106972 A1 & EP 1886704 A1 & KR 10-2007-0116667 A & CN 101151056 A | 1-7 |
| A | JP 2012-5827 A (TORAY INDUSTRIES, INC.) 12 January 2012, entire text, all drawings (Family: none) | 1-7 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 November 2018 (29.11.2018) | 11 December 2018 (11.12.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2018/033129

C (Continuation).   DOCUMENTS CONSIDERED TO BE RELEVANT

| Category* | Citation of document, with indication, where appropriate, of the relevant passages | Relevant to claim No. |
|---|---|---|
| A | JP 7-204265 A (SEKISUI CHEMICAL CO., LTD.) 08 August 1995, entire text, all drawings (Family: none) | 1-7 |
| A | JP 60-252423 A (ASAHI KASEI INDUSTRY CO., LTD.) 13 December 1985, entire text, all drawings & US 4839290 A & EP 147689 A2 | 1-7 |
| A | JP 56-130160 A (TERUMO CORP.) 12 October 1981, entire text, all drawings (Family: none) | 1-7 |

Form PCT/ISA/210 (continuation of second sheet) (January 2015)

**EP 3 679 964 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 60193468 A
- WO 2008038785 A
- WO 2012133399 A